Europäisches Patentamt

European Patent Office

Office européen des brevets

(11) Publication number: **0 657 151 A1**

# EUROPEAN PATENT APPLICATION

(21) Application number: **94307888.1**

(51) Int. Cl.6: **A61F 9/00**

(22) Date of filing: **26.10.94**

(30) Priority: **12.11.93 US 151726**

(43) Date of publication of application:
**14.06.95 Bulletin 95/24**

(84) Designated Contracting States:
**CH DE FR GB IT LI**

(71) Applicant: **INTELLIGENT SURGICAL LASERS, INC.**
**4520 Executive Drive No. 1**
**San Diego,**
**California 92121-3028 (US)**

(72) Inventor: **Juhasz, Tibor**
**22 Newton Court**
**Irvine,**
**California 92715 (US)**
Inventor: **Bille, Josef F.**
**Hermann Lunsweg 44/1**
**D-6900 Heidelberg (DE)**

(74) Representative: **Coxon, Philip et al**
**Eric Potter & Clarkson**
**St. Mary's Court**
**St. Mary's Gate**
**Nottingham NG1 1LE (GB)**

(54) **Intrastromal photorefractive keratectomy.**

(57) A method for performing intrastromal photorefractive keratectomy in the cornea (12) of an eye, using a pulsed laser beam, includes the initial step of focusing the beam to a spot size at a start point (42) in the stroma (22). The start point is located in the stroma and at a predetermined distance behind the epithelium (18) of the cornea. Beginning with the start point, a volume of stromal tissue which is substantially equal to the volume of the spot size is disrupted by the laser beam. Importantly, the photodisruption is accomplished using an irradiance for the beam which is less than ten times greater the threshold for optical breakdown of the stromal tissue. Subsequently, the beam is focused in a patterned sequence to spot sizes at other discrete points in the stroma. At each point the stromal tissue is photodisrupted. With this progressive pattern of photodisruption, each spot is placed substantially adjacent a volume of previously disrupted tissue. The resultant photodisrupted tissue creates a layer (52) which is substantially centro-symmetrical around the optical axis and in which all points in the layer are substantially equidistant from the epithelium. A plurality of layers (52,54,56) can be created.

Fig. 2

## FIELD OF THE INVENTION

The present invention relates to a method of using a pulsed laser to alter visually transparent matter.

## BACKGROUND OF THE INVENTION

It is known that the cornea of an eye can, in certain instances, be surgically reshaped to correct and improve vision. Indeed, as more fully set forth below, there are several different types of ophthalmic surgical procedures which can be employed for this purpose. Although the types of procedures may vary, the ultimate object is the same. Namely, the object is to reduce the thickness of the cornea so that it properly refracts light entering the eye for subsequent focusing on the retina of the eye.

A well known surgical operation for reshaping the cornea of an eye is the procedure known as radial keratotomy. This procedure, which is used primarily to correct myopia, is performed by making a series of radial incisions on the surface of the cornea. These incisions extend from the outer edge of the cornea toward its center in spoke-like fashion to weaken selected sections of the cornea. With these weakened sections, the fluid pressure of the aqueous humor inside the eye will cause the cornea to deform. As intended for this procedure, the desired deformation is a flattening of the cornea to provide proper light refraction for improved vision.

In recent years, the use of cutting tools to make incisions into the cornea for vision corrections is gradually being replaced or supplemented by the use of new surgical procedures using laser beams. Rather than making incisions, laser beams which reshape the cornea do so by actually removing corneal tissue. This is accomplished by a process which is generally known as photoablation. Heretofore, the photoablation of corneal tissue has been accomplished primarily by focussing laser beams onto the exposed anterior surface of the eye. The result which can be achieved is dependent on two interrelated factors. First, the particular laser system which is employed to generate a laser beam will significantly affect how the photoablation process can be accomplished. Second, the method by which the laser beam is manipulated to accomplish photoablation will effectively determine the efficacy of the procedure.

For ophthalmic laser systems, several different types of laser beams have been suggested. For example, U.S. Patent No. 4,665,913 which issued to L'Esperance, Jr. for an invention entitled "Method for ophthalmological Surgery" discloses a corneal reshaping procedure using an excimer laser. As another example, U.S. Patent No. 4,907,586 which issued to Bille et al. for an invention entitled "Method for Reshaping the Eye", and which is assigned to the same assignee as the present invention, discloses a corneal reshaping procedure which uses a pulsed laser beam.

Although using laser beams for the removal of corneal tissue from the anterior surface of the cornea is known to be effective, the superficial removal of tissue requires photoablation of several layers of different tissues in the cornea. These include portions of the epithelium, Bowman's membrane and the stroma. The present invention recognizes that it is preferable to limit this activity to only one type of tissue, namely the stroma. Further, the present invention recognizes that internal tissue photoablation, or more precisely "photodisruption", can be effectively accomplished using a pulsed laser beam if the irradiance of the beam, and its spot size, are effectively controlled.

In light of the above, it is an object of the present invention to provide a method for altering visually transparent matter using a pulsed laser beam which controls the irradiance of the laser beam to limit the amount of matter which is subject to photodisruption. Another object of the present invention is to provide a method which controls the stop size and spot configuration of the laser beam to permit alteration of transparent matter by contiguous photodisruption at successively adjacent spots. Still another object of the present invention is to provide a method which removes matter in a predetermined pattern to attain a desired result. Yet another object of the present invention is to provide a method for altering visually transparent matter which is relatively easy to perform and which is comparatively cost effective.

## SUMMARY OF THE INVENTION

In accordance with the present invention, a method for altering visually transparent matter uses a pulsed laser beam which is sequentially focused to individual spots at a plurality of points in the matter. Photodisruption of the matter occurs at each spot where the beam is focused, and the volume of matter disrupted at each spot is approximately equal to the volume of the spot size.

The present invention therefore provides a method for using a pulsed laser beam to alter visually transparent matter which comprises the steps of: focusing said beam to a spot at a start point in said matter; disrupting said matter in a volume approximately equal to the volume of said spot; refocusing said beam to a spot at a point in said matter, said refocused spot being substantially adjacent said volume of said disrupted matter; subsequently disrupting said matter in a volume approximately equal to the volume of said refocused spot; and repeating said refocusing and said subsequently disrupting steps to disrupt said matter in a predetermined pattern.

The physical characteristics of the laser beam as well as the manner of focusing the laser beam are important to the proper performance of the method of the present invention. As indicated above, these considerations are interrelated.

First, insofar as the physical characteristics of the laser beam are concerned, several factors are important. Preferably, the wavelength of the laser beam should be in the range of 3-0.3 $\mu$m. Also, the irradiance of the beam for accomplishment of photodisruption of the matter at the spots should be greater than the threshold for optical breakdown of the tissue. The irradiance is conveniently approximately of the order of 200 GW/cm$^2$. In any event, the irradiance of the laser beam should preferably not be more than ten times greater than the threshold for optical breakdown of the matter and, as a practical matter, most preferably not more than one hundred times greater than the threshold. Further, the pulse repetition frequency of the pulsed laser beam is preferably in the range of approximately 0.1 to 100 KHz and preferably in the range of 1-10 KHz.

Second, insofar as the focusing of the laser beam is concerned, spot size and spot configuration are important. The spot size of the focused laser beam should be small enough to achieve optical breakdown of the matter in the desired spot. Typically, this requires the spot size be less than 100 $\mu$m in diameter. Additionally, it is preferable that the spot be as close to spherical as possible. To achieve this configuration for the spot, it is necessary that the laser beam be focused from a relatively wide angle cone. For the present invention, the cone angle will preferably be in the range of 15°-45°.

To perform the method in accordance with the invention, the laser beam is first focused to a spot at a start point in the matter. The laser beam is then activated and matter at the start point is photodisrupted. Importantly, because spot size/configuration and the irradiance level of the laser beam are closely controlled for the present invention, the volume of matter which is photodisrupted is carefully maintained. Preferably, the diameter of this volume is about the same as the volume occupied by the spot. It is noted, however, that during photodisruption of the matter, a cavitation bubble may result which preferably has a volume with a diameter which is about twice the diameter of the spot. Next, the laser beam is focused to a spot at another point in the matter which is substantially adjacent the volume of previously photodisrupted matter. Again, the laser beam is activated and matter at the new spot is photodisrupted to add to the total volume of matter which had previously been photodisrupted. This process is continued, proceeding from point to point through the matter, until all of the matter to be affected has been photodisrupted.

It is preferably that photodisruption of the transparent matter be accomplished at a plurality of adjacent points in a patterned sequence, conveniently a spiral pattern. The result is a layer of photodisrupted matter. In accordance with the present invention, a plurality of superposed photodisrupted layers can be created.

## BRIEF DESCRIPTION OF THE DRAWINGS

The method of the invention will now be described for illustrative purposes only by reference to a method for intrastromal photorefractive keratectomy, with reference to the accompanying drawings, taken in conjunction with the accompanying description, in which similar reference characters refer to similar parts, and in which:

Figure 1 is a cross sectional view of the cornea of an eye shown in relationship to a schematically depicted laser unit;

Figure 2 is a cross sectional view of the cornea of an eye showing the anatomical layers thereof;

Figure 3 is a schematic representation of the positioning of adjacent laser beam spots and the resultant sequential disruption of stromal tissue which occurs during implementations of the method of the present invention; and

Figure 4 is a plan view schematic representation of a predetermined pattern and the resultant layer in which stromal tissue is photodisrupted by implementation of the method the present invention.

## DESCRIPTION OF THE PREFERRED EMBODIMENTS

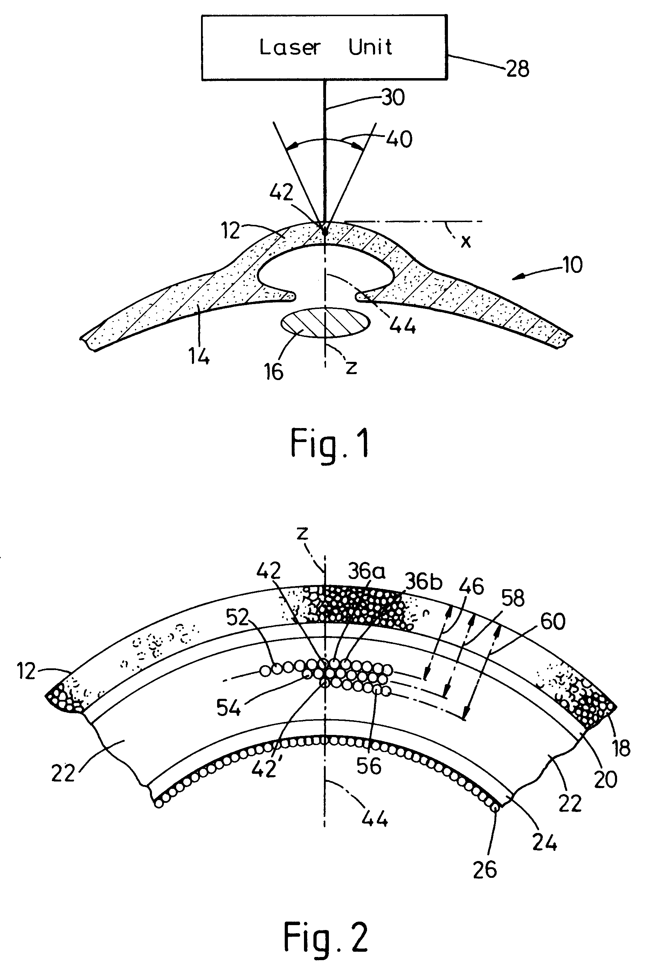

Referring initially to Figure 1, a cross section of part of an eye is shown and generally designated 10. For reference purposes, the portion of eye 10 which is shown includes the cornea 12, the sclera 14 and the lens 16. Further, in accordance with standard orthogonal ocular referencing coordinates, the z-axis of z direction is generally oriented on the optical axis of the eye 10.

Consequently, the x and y directions establish a plane which is generally perpendicular to the optical axis.

As best seen in Figure 2, the anatomy of the cornea 12 of an eye 10 includes five different identifiable tissues. The epithelium 18 is the outermost tissue on the exterior of the cornea 12. Behind the epithelium 18, and ordered in a posterior direction along the z-axis, are Bowman's membrane 20, the stroma 22, Descemet's membrane 24, and the endothelium 26. Of these various tissues, the region of most interest to the present invention is the stroma 22.

Returning for the moment to Figure 1, it will be seen that the method of the present invention incorporates a laser unit 28 which must be capable of generating a pulsed laser beam 30 having certain characteristics. Importantly the pulsed laser beam 30 should be monochromatic light having a wavelength ($\lambda$) which is transparent to all tissues of the cornea 12. Preferably, wavelength ($\lambda$) of laser beam 30 will be in the range of from one to three hundred and fifty microns ($\lambda = 3\text{-}0.3\ \mu m$). Also, the pulse repetition rate of laser beam 30 should be approximately in the range of from one hundred Hertz to one hundred thousand Hertz (0.1-100 KHz). An additional factor of great importance to the present invention is that the irradiance of laser beam 30 must be circumscribed and well defined. The main concern here is that the irradiance of beam 30 will, in large part, determine the photodisruptive capability of pulsed laser beam 30 on tissue of the stroma 22.

Irradiance, or radiant flux density, is a measure of the radiant power per unit area that flows across a surface. As indicated by the following expression, the irradiance of laser beam 30 is a function of several variables. Specifically:

$$\text{Irradiance} = \frac{(\text{pulse energy})}{(\text{pulse duration})(\text{spot size})}$$

From the above expression for irradiance it can be appreciated that, for a constant level of irradiance, the irradiance is proportional to the amount of energy in each pulse of beam 30. On the other hand, irradiance is inversely proportional to pulse duration and spot size. The significance of this functional relationship stems from the fact that the irradiance of pulsed laser 30 should be approximately equal to the optical breakdown threshold for stromal tissue 22. This threshold is known to be about two hundred gigawatts per square centimeter (200 GW/cm$^2$). Insofar as each factor's contribution to irradiance is concerned, it is important to recognize that no one factor can be considered individually. Instead, the pulse energy, pulse duration and spot size of laser beam 30 are interrelated and are each variable.

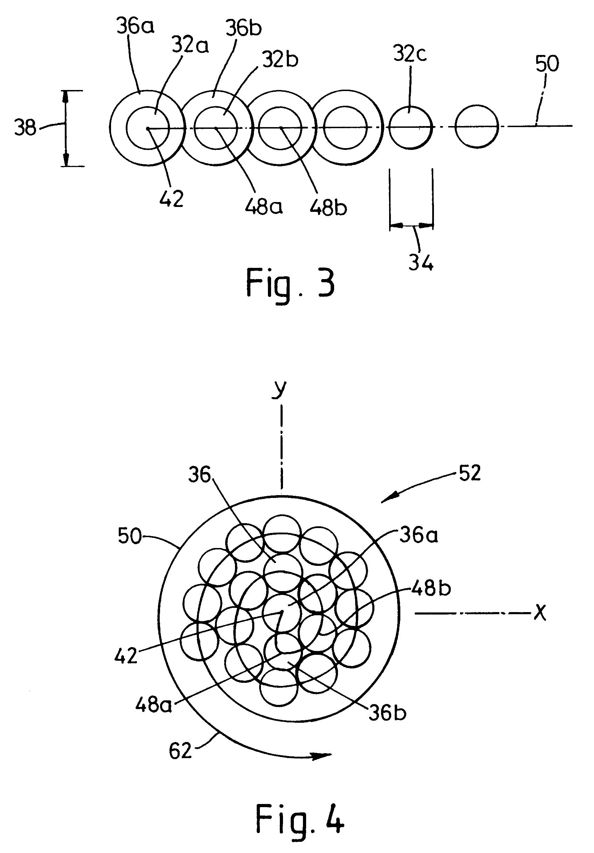

For purposes of the present invention, the pulse duration of pulses in laser beam 30 are preferably in the range of from one hundred femto seconds to ten nanoseconds, and preferably in the range of one to one hundred pico seconds (1-100 psec). As for the spot size to which each pulse is focused, the determinative consideration is that the spot size should be small enough to achieve optical breakdown in a volume of stromal tissue 22 which is approximately equal to the volume of the spot size. This relationship is perhaps best seen in Figure 3.

In Figure 3, a succession of spot sizes 32 are shown. All spot sizes 32 are substantially spherical, or slightly ellipsoidal, and have substantially the same volume. As such, they can each be characterized as having a diameter 34. Figure 3 also shows the general relationship between each spot size 32 and the cavitation bubble 36 which results when laser unit 28 is activated to irradiate a spot size 32. The cavitation bubble 36, like the spot size 32, will be generally spherical and can be characterized by a diameter 38. As indicated above, preferably, diameter 38 of cavitation bubble 36 is the same as the diameter 34 of the corresponding spot size 32. This, however, cannot always be achieved. In any event, it is important that the volume of cavitation bubble 36 not be significantly larger than the volume of the spot size 32. For the present invention, it is preferable that the diameter 34 of spot size 32, and thus the volume of removed tissue, be less than about one hundred microns (<100$\mu m$), and that the diameter 38 of cavitation bubble 36 be no more than about twice the length of diameter 34 of spot size 32.

As indicated above, the spot size 32 is substantially spherical. To configure spot size 32 as close as possible to a sphere, rather than as an elongated ellipsoid, it is necessary for laser beam 30 to be focused through a rather wide cone angle 40 (see Figure 1). For purposes of the method of the present invention, cone angle 40 should be in the range of from fifteen to forty five degrees (15°-45°). Presently, the best results are known to be achieved with a cone angel of about thirty six degrees (36°).

For the practice of the method of the present invention, it is first necessary for the physician to somehow stabilize the eye 10. After the eye 10 has been stabilized, laser beam 30 is focused to a spot size 32 at a start point 42 in the stroma 22. Specifically, for many procedures, the start point 42 is located generally on the z-axis 44 at a distance 46 which is approximately eighty microns ($80\mu$m) behind the epithelium 18. As used here, behind means in a posterior direction or inwardly from the epithelium 18. Once laser beam 30 is so focused, the laser unit 28 is activated to irradiate the spot size 32 at start point 42. The result is that a volume 36 of stromal tissue 22 is disrupted and removed from the stroma 22.

The physical consequences of photodisruption of stromal tissue 22 at the start point 42, and at other points 48 in the stroma 22, are manifold. Some tissue around the start point 42 is, of course, removed. Additionally, however, by-products such as carbon dioxide ($CO_2$), carbon monoxide (CO), nitrogen ($N_2$) and water ($H_2O$) are formed. As stated above these by-products create a cavitation bubble 36 in the tissue of stroma 22.

As indicated in Figure 3, once the cavitation bubble 36 has been created, the laser beam 30 is repositioned for refocussing at another point 48. In Figure 3 it is shown that the refocused point 48a is substantially adjacent start point 42 and that both the point 48a and start point 42 lie on a path 50. Importantly, the distance along path 50 between start point 42 and adjacent point 48a is preferably less than the diameter 38 of disrupted tissue volume. This is so in order that the sequential volumes of disrupted tissue in stroma 22 will overlap. In effect, the size of the cavitation bubble 36 of disrupted tissue volume will determine the separation distance between points 42, 48 along the path 50. As implied here, subsequent points 48b et seq. will also lie on the predetermined path 50 and the disrupted tissue volume at any respective point 48 will overlap with the volume of tissue previously disrupted in stroma 22. Consequently, the separation distance between points 48 on path 50 must be established so that tissue removal along the path 50 will be contiguous.

Referring back to Figure 1, it will be seen that a plurality of disrupted tissue volumes along the path 50 can be juxtaposed to establish a contiguous layer 52 of disrupted stromal tissue 22. Importantly, each disrupted tissue volume in the layer 52 is equidistant from the surface of the epithelium 18. It is important to note that the layer 52 should be created close to the epithelium 18. This is so because by creating layer 52 close to the epithelium 18, vision correction will be more effective. Nevertheless, there are limitations as to how close layer 52 can be to the epithelium 18 in order to avoid unwanted photodisruption of Bowman's membrane 20 and the epithelium 18. Accordingly, no point 48 in the layer 52 should be closer than approximately eighty microns ($80\mu$m) to the anterior surface of epithelium 18. With these constraints in mind, the layer 52 created by laser beam 30 will be completely within the stroma 22 and will be somewhat curved to conform to the outer anterior surface of epithelium 18.

It is known that at a depth of approximately eighty microns ($80\mu$m) behind the epithelium 18, the photodisruption of a layer 52 will result in a thinning of the cornea 12 by approximately ten to twenty microns ($10$-$20\mu$m). Recall, however, that the preferred diameter of a spot size 32 may be as much as one hundred microns. The discrepancy between this dimension of the spot size 32 and the resultant thinning of cornea 12, which is considerably less, is due to the fact that after there has been photodisruption and the creation of the cavitation bubble of disrupted tissue volume 36, the volume 36 will eventually collapse. Even so, this thinning of cornea 12 corresponds to a vision correction of approximately one to two diopters. Unfortunately, this may not be enough correction. Consequently, additional layers of disrupted tissue may need to be created.

As shown in Figure 2, a plurality of layers can be created in stroma 22 by the method of the present invention. Figure 2 shows a layer 54 which is located behind the layer 52 and a layer 56 which is located behind the layer 54. Indeed, as many as five or six layers can be so created, with each layer causing an additional correction of approximately one or two diopters.

Whenever a plurality of layers are to be created, it is important that the most posterior layer be created first, and that each successive layer be created more anterior and closer to the epithelium 18 than any previously created layers. For example, to create layers 52, 54 and 56 which are respectively at distances 46, 58 and 60 behind the epithelium 18, it is necessary to start first with the creation of the layer 56. Then, in order, layers 54 and 52 can be created. Also, because it is anticipated that each layer will effectively remove approximately only fifteen microns of tissue, it is necessary that the layer 56 be created thirty microns behind layer 52. Recall, however, that layer 52 should eventually be located about eighty microns behind the epithelium 18. Thus, the first point to be photodisrupted for layer 56 will be located on the z-axis 44 at a distance 60 behind epithelium 18 which is equal to approximately one hundred and ten microns. As was disclosed above regarding layer 52, all points in layer 56 will be approximately at a distance 60, and thus equidistant, from the epithelium 18. The distance 58 for layer 54 will then be approximately ninety five microns and, finally, distance 46 for layer 52 will be the required eighty microns.

Figure 4 shows a plan view of the layer 52 as seen looking toward the eye 10 along z-axis 44. Also, Figure 4 shows that the start point 42 and the sequence of subsequent points 48, as in Figure 3, all lie along the path 50. Further, Figure 4 shows that the path 50 can be set as a pattern 62 and, as shown in

EP 0 657 151 A1

Figure 4, this pattern 62 can be a spiral pattern. It is to be appreciated that the spiral pattern 62 can be extended as far as is desired and necessary to create the layer 52 of disrupted tissue volumes 36. It is also to be appreciated that the final pattern 62 will be approximately centro-symmetric with respect to the optical axis (z-axis 44) of the eye 10.

**Claims**

1. A method for using a pulsed laser beam to alter visually transparent matter which comprises the steps of:

    focusing said beam to a spot at a start point in said matter;

    disrupting said matter in a volume approximately equal to the volume of said spot;

    refocusing said beam to a spot at a point in said matter, said refocused spot being substantially adjacent said volume of said disrupted matter;

    subsequently disrupting said matter in a volume approximately equal to the volume of said refocused spot; and

    repeating said refocusing and said subsequently disrupting steps to disrupt said matter in a predetermined pattern.

2. A method according to claim 1 wherein said disrupting steps are accomplished with said beam having an irradiance less than one hundred times greater than the threshold for optical breakdown of said matter at said spots.

3. A method according to claim 1 or 2 wherein the size of each of said spot is less than one hundred microns in diameter (<100 $\mu$m).

4. A method according to any one of the preceding claims wherein the wavelength of light in said beam is in the range of between three and three tenths microns (3-0.3 $\mu$m).

5. A method according to any one of the preceding claims wherein the pulse frequency of said beam is in the range of from one hundred Hertz to one hundred thousand Hertz (0.1-100 KHz).

6. A method according to any one of claims 2 to 5 wherein said irradiance is approximately two hundred gigawatts per square centimeter (200 GW/cm$^2$).

7. A method according to any one of the preceding claims wherein the diameter of said matter disrupted by each of said spots is approximately equal to the diameter of the volume of respective said spots.

8. A method according to any one of the preceding claims wherein said pattern creates a layer of disrupted matter.

9. A method according to claim 8 further comprising the step of repeating said steps of claim 1 to create a plurality of layers.

10. A method according to any one of the preceding claims wherein said pattern is a spiral pattern.

11. A method according to any one of the preceding claims wherein said beam is focused and refocused with a cone angle in the range of from fifteen to forty five degrees (15°-45°).

12. A method according to any one of the preceding claims wherein said pulse of said pulsed laser beam has a deviation in the range of approximately one to forty picoseconds (1-40 psecs).

6

Fig. 1

Fig. 2

Fig. 3

Fig. 4

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | US-A-4 976 709 (SAND)<br>* column 7, line 46 - column 8, line 46; claims * | 1-12 | A61F9/00 |
| A | EP-A-0 484 005 (SUMMIT TECHNOLOGIE, INC.)<br>* column 4, line 22 - column 5, line 3; claims 1,8; figure 2 * | 1-12 | |
| A | WO-A-89 06519 (REFRACTIVE LASER RESEARCH & DEVELOPMENT PROGRAM, LTD.)<br>* page 13, line 24 - page 14, line 28; claims 1-9,48-59 * | 1-12 | |
| D,A | US-A-4 907 586 (BILLE ET AL.)<br>* column 10, line 30 - line 54; claims; figure 5 * | 1-12 | |
| P,A | WO-A-94 09849 (SWINGER)<br>* page 37, line 12 - page 39, line 29; figure 18 * | 1-12 | |
| | | | TECHNICAL FIELDS SEARCHED (Int.Cl.6) |
| | | | A61F |

The present search report has been drawn up for all claims

| Place of search | Date of completion of the search | Examiner |
|---|---|---|
| BERLIN | 3 March 1995 | Kanal, P |